# EUROPEAN PATENT SPECIFICATION

(11) **EP 1 675 656 B1**
(45) Date of publication and mention of the grant of the patent: **16.11.2011**
(21) Application number: 04765497.5
(22) Date of filing: 20.09.2004
(51) Int. Cl.: A61K 8/20

(54) **COSMETIC COMPOSITIONS FOR IMPARTING SKIN RADIANCE**
KOSMETISCHE ZUSAMMENSETZUNGEN ZUR VERLEIHUNG VON STRAHLENDEM AUSSEHEN FÜR DIE HAUT
COMPOSITIONS COSMETIQUES AMELIORANT L'ECLAT DE LA PEAU

(30) Priority: 09.10.2003 US 682657
(43) Date of publication of application: 05.07.2006
(73) Proprietor: Unilever PLC, London EC4Y 0DY (GB); Unilever N.V., 3013 AL Rotterdam (NL)
(72) Inventor: POLONKA, Jack, Edgewater, NJ 07020 (US)
(74) Representative: Acham, Nicholas Clive
(86) International application number: PCT/EP2004/010633
(87) International publication number: WO 2005/039522

(56) References cited:
- US-A- 5 000 937
- US-B1- 6 174 533
- PATENT ABSTRACTS OF JAPAN vol. 2000, no. 20, 10 July 2001 (2001-07-10) & JP 2001 058925 A (KOSE CORP), 6 March 2001 (2001-03-06)
- PATENT ABSTRACTS OF JAPAN vol. 2002, no. 02, 2 April 2002 (2002-04-02) & JP 2001 288039 A (SHISEIDO CO LTD), 16 October 2001 (2001-10-16)
- PATENT ABSTRACTS OF JAPAN vol. 1995, no. 07, 31 August 1995 (1995-08-31) & JP 7 101828 A (TORAY IND INC), 18 April 1995 (1995-04-18)

## Description

### FIELD OF THE INVENTION

The invention relates to skin care and/or cleansing compositions having flat platy crystalline particles and providing skin radiance.

### BACKGROUND OF THE INVENTION

"(S)he looks radiant!" is a comment consumers would take as a complement. Skin radiance is lost with age and consumers seek a cosmetic skin care product that would restore or improve the appearance of skin radiance whilst maintaining natural skin tone or color. Specifically, such products should, upon application to the skin: 1) provide a radiant appearance to skin and 2) provide a colorless or natural skin finish.

Trends in cosmetics have been to use materials, such as mica, bismuth oxychloride, boron nitride, titanium dioxide, etc., as pigments in nail polish, lipstick, eye shadow, and other cosmetics requiring that the natural skin tone and color be masked. However, these masking cosmetics are not suitable for imparting appearance of radiance and natural glow to the skin. Therefore, it was clear that further work was necessary in order to develop a skin care and/or cleansing product that would perform to the criteria of radiance and natural glow.

### SUMMARY OF THE INVENTION

The shortcomings of the prior art are overcome with compositions that can provide the consumer-desired optical properties of radiance, without opacifying natural skin color. The present invention includes a skin care or cleansing composition comprising:
a) 0.01 % to 1 % by weight of said composition of solid single-crystal, flat, plate-like particles; said particles having an index of refraction of 1.8 to 2.2; and
b) a cosmetically acceptable vehicle;
wherein said composition has an opacity of less than about 20%;
wherein said plate-like particles have a particle diameter of 10 to 30 microns;
wherein said plate-like particles are bismuth oxychloride, and wherein opacity is measured using a Hunterlab LabScan XE automated spectrophotometer with composition coatings prepared on Leneta Form 2A opacity test charts held in place on a vacuum plate, an 8-path wet film applicator used to coat a film with a wet thickness of 50.8 µm and the coatings air dried before opacity measurement.

The plate-like particles have a particle diameter of 10 to 30 microns, and a particle thickness of 0.1 to 5 microns. The plate-like particles comprise 0.05 % to 0.5 % by weight of the skin care and/or cleansing composition, preferably, 0.1 % by weight of the composition. The plate-like particles may be present in a polar solvent prior to incorporation in the composition.

The composition may further contain skin benefit agents, which, when present, are in an amount of at least 0.0001% by weight of the composition.

Skin benefit agents optionally include retinoids, essential fatty acids, alpha-hydroxy carboxylic acids, beta-hydroxy carboxylic acids, poly-hydroxy carboxylic acids, and mixtures thereof. Examples of alpha-hydroxy carboxylic acids are glycolic acid, lactic acid, 2-hydroxyoctanoic acid, and mixtures thereof. An example of beta-hydroxy carboxylic acid is salicylic acid. Other skin benefit agents include ferulic acid and sebacic acid.

The present invention also includes a method of imparting skin radiance without coloring or opacifying, by applying to the skin the inventive composition.

Except in the operating and comparative examples, or where otherwise explicitly indicated, all numbers in this description indicating amounts of material or conditions of reaction, physical properties of materials and/or use are to be understood as modified by the word "about." All amounts are by weight of the final composition, unless otherwise specified.

As used herein, the term "comprising" means including, made up of, composed of, consisting and/or consisting essentially of.

The term "leave-on" as used with reference to compositions herein means a composition that is applied to or rubbed on the skin, and left thereon.

The term "skin" as used herein includes the skin on the face (except eye lids and lips), neck, chest, abdomen, back, arms, hands, and legs.

The term "radiance" as used herein means skin glow while maintaining natural looking skin or skin tone, i.e. appearance of natural skin glow.

The term "solid" as used herein means that the material is not fluid at 25°C.

The term "wash-off" as used with reference to compositions herein means a skin cleanser that is applied to or rubbed on the skin and rinsed off substantially immediately subsequent to application.

### DETAILED DESCRIPTION OF THE INVENTION

The inventive compositions and products uniquely create a "radiant" effect on skin without producing a cosmetic appearance. In order to provide the consumer-desired optical properties of skin radiance, the skin care and/or cleansing compositions of the present invention include a skin care or cleansing composition comprising:
a) 0.01 % to 1 % by weight of said composition of solid single-crystal, flat, plate-like particles; said particles having an index of refraction of 1.8 to 2.2; and
b) a cosmetically acceptable vehicle;
wherein said composition has an opacity of less than about 20%;
wherein said plate-like particles have a particle diameter of 10 to 30 microns;
wherein said plate-like particles are bismuth oxychloride, and wherein opacity is measured using a Hunterlab LabScan XE automated spectrophotometer with composition coatings prepared on Leneta Form 2A opacity test charts held in place on a vacuum plate, an 8-path wet film applicator used to coat a film with a wet thickness of 50.8 µm and the coatings air dried before opacity measurement.

The particles are single-crystal and plate-like, such that upon application to skin, the particles impart a natural radiance to the skin.

The product is designed to impart radiance while maintaining natural skin tone, which is achieved by controlling the light transmission properties, or opacity, of the product composition.

The composition may further contain skin benefit agents. Skin benefit agents for the purposes of the present invention include retinoids, essential fatty acids, alpha-hydroxy carboxylic acids, beta-hydroxy carboxylic acids, poly-hydroxy carboxylic acids, and mixtures thereof. Examples of alpha-hydroxy carboxylic acids are glycolic acid, lactic acid, 2-hydroxyoctanoic acid, and mixtures thereof. An example of a beta-hydroxy carboxylic acid is salicylic acid.

The present invention also includes a method of imparting radiant skin appearance, by applying to the skin the inventive composition.

### Single-Crystal, Flat, Plate-like Particles

The inventive compositions employ solid particles that are single-crystal, flat and plate-like, to impart a radiant appearance to the skin upon application. By virtue of being flat single crystals, the particles deliver high reflectance. Flat plate-like crystals can generate this natural radiant appearance through optical reflectance. The ideal properties for the material would be a plate-like crystal habit with dimensions of about 10 to about 30 microns in diameter; about 0.1 to about 5 microns thick, preferably about 0.1 to about 0.5 microns thick; a smooth surface; and an index of refraction of about 1.8 to about 2.2.

Size of the plate-like particles is important because smaller particles reflect too little light to be readily apparent, while larger particles would be visible as discrete objects and thereby provide too much glitter or reflectance. The reflectance (index of refraction) of the plate-like crystal cannot be too high. Too high an index of refraction will inhibit the transmission of natural skin color and create a cosmetic sheen. With too low an index of refraction, the particles will have approximately the same index of refraction as the skin or the product film, resulting in a weak reflectance, thereby diminishing the appearance of radiance.

A single-crystal structure is also key because the smoothness of the crystal surface minimizes opacity or diffuse scattering effects, which would lead to an artificial cosmetic effect. A single-crystal structure maximizes the smooth crystalline surface area. As the facet of a crystal is the smoothest surface possible, it maximizes the degree of reflectance while minimizing the opacity. The product is designed to impart radiance while maintaining natural skin tone, which is achieved by controlling the opacity of the composition.

Bismuth oxychloride is sold under the RONA Biron brand from EMD Chemicals Inc., Hawthorne, New Jersey.

The inventive compositions contain about 0.01 % to about 1 % of the solid particles, preferably about 0.05 % to about 0.5 %, and more preferably about 0.1 %, to obtain the best appearance of radiance while maintaining a natural look. The exact amount depends on the final composition and the nature of the other ingredients in the composition.

In the inventive compositions, the solid single crystal plate-like particles are, preferably, dispersed in a polar solvent. If skin benefit agents are included, they are employed in such amounts as to provide a desired skin benefit and yet to not compromise the appearance of radiance.

### Index of Refraction of Plate-like Particles

The index of refraction is a measure of the ability of a substrate to bend light incident thereon, and is well known to one skilled in the art. The index of refraction herein is a measure of optical properties the inventive compositions are engineered to achieve, i.e., shine, reflectance, transmission, radiance, glow. Ideally, the index of refraction is about 1.8 to about 2.2. The exemplary suitable materials: bismuth oxychloride, aluminum oxide, zirconium oxide and boron nitride fall within this range; while, in contrast, materials such as mica (1.57), glass (1.5-1.6), titanium dioxide (2.5-2.7) and iron oxides (3.1) lie outside the suitable range of index of refraction.

### Opacity of the Composition

The compositions according to the present invention are substantially translucent, so as to be consistent with a natural skin appearance, whilst imparting radiance thereto. Opacity is a measurement of translucency or opacity.

The inventive skin care and/or cleansing compositions have an opacity of less than about 20 %; preferably less than about 10 %; most preferably, in order to obtain the most desired natural skin tone or color, less than about 5 %. The opacity of the composition must be in proper balance with the index of refraction of the plate-like particles therein.

### Opacity Measurement Protocol

A Hunterlab LabScan XE automated spectrophotometer was used to measure the opacity of product coatings. The coatings were prepared on Leneta Form 2A opacity test charts held in place on a vacuum plate, and an 8-path wet film applicator was used to coat a film with a wet thickness of 2 mils, i.e. 50.8 µm (all equipment supplied by Paul N. Gardner Co.). This wet film thickness was chosen by applying 75 µL of composition on about 2 in.², i.e. 1290 mm², which corresponds to a wet film thickness of 58 µm. The coatings were air dried before the opacity measurement.

The opacity values were reported as percent opacity, defined as the Y value of the coating on the black area of the test chart divided by the Y value on the white area times 100%. The Y value is the CIE Tristimulus Y coordinate measured by the Hunterlab instrument. If a coating were fully transparent, the opacity would be 0%; if fully opaque, it would be 100%.

### Skin Benefit Agents

Skin benefit agents may also be optionally, but preferably, included in the compositions of the present invention. Skin benefit agents are defined as active skin benefit agents other than emollients and other than ingredients that merely improve the physical characteristics of the composition.

Examples of skin benefit agents include retinoids, essential fatty acids, alpha-hydroxy acids, beta-hydroxy acids, polyhydroxy acids, skin lightening agents, and mixtures thereof. Specific examples of skin benefit agents include retinol, retinoic acid, glycolic acid, lactic acid, 2-hydroxyoctanoic acid, salicylic acid, ferulic acid and sebacic acid, or combinations thereof.

A preferred optional ingredient is selected among essential fatty acids (EFAs). EFAs are fatty acids which are essential for the plasma membrane formation of all cells, in keratinocytes EFA deficiency makes cells hyperproliferative. Supplementation of EFA corrects this. EFAs also enhance lipid biosynthesis of epidermis and provide lipids for the barrier formation of the epidermis. The essential fatty acids are preferably chosen from linoleic acid, Υ-linolenic acid, homo-Υ-linolenic acid, columbinic acid, eicosa-(n-6,9,13)-trienoic acid, arachidonic acid, timnodonic acid, hexaenoic acid and mixtures thereof.

When present, the amount of the skin benefit agent is at least about 0.0001% by weight of the composition.

### Other Optional Skin Benefit Materials and Cosmetic Adjuncts

Hydrophobically modified polymeric emulsifiers may be optionally present in the inventive compositions as a co-structurant, typically with a trade name, Pemulen TR series, from 0.001 to 2% by weight, supplied by BF Goodrich Co., Cleveland, OH.

pH adjusting agents may be used to maintain the desired pH, if necessary, especially in the presence of certain acidic actives which may significantly lower the pH of the compositions. Preferred pH adjusting agents include inorganic or organic bases such as ammonium hydroxide, potassium hydroxide, sodium hydroxide and triethanolamine. Preferred pH adjusting agents also include inorganic acids such as hydrochloric acid.

Emollient materials (fluid oils) selected from the groups of silicone oils or synthetic esters may be incorporated into the compositions of the present invention. Oily sunscreens, when used in the composition are considered to be emollient materials, and will be further discussed below.

Silicone oils may be included in the compositions as emollient materials. These are preferably chosen from cyclic or linear polydimethylsiloxanes containing from about 3 to about 9, preferably from about 4 to about 5, silicon atoms. Other silicone oils may be also included, such as polyalkyl siloxanes, polyalkylaryl siloxanes and polyether siloxane copolymers (e.g. dimethicone copolyol). The polyalkyl siloxanes useful herein include, for example, polydimethyl siloxanes with viscosities of from about 5 to about 100,000 centistokes at 25°C, preferably, polydimethyl siloxanes having viscosities from about 10 to about 400 centistokes at 25°C. The oils may be employed singly or in mixtures with one another.

Suitable ester emollients include: esters of fatty acids or alcohols and hydrocarbons, preferably C₈-C₂₀ alkyl ester of fatty acids such as , isopropyl myristate, isopropyl palmitate, isostearyl palmitate, tridecyl salicylate, C₁₂-₁₅ octanoate and isopropyl stearate, or any mixtures thereof.

The inventive compositions most preferably further include an ingredient selected from the group consisting of antioxidants, reducing agents, chelating agents, and mixtures thereof to improve the stability of the cosmetic cream. These ingredients provide an additional level of protection against oxidation of skin benefit agents in the cosmetic cream. Common examples of antioxidants, reducing agents and chelating agent for the present formulations can be found in the CTFA International Cosmetic Ingredient Dictionary 4th Edition, The Cosmetic, Toiletry, and Fragrance Association, Inc., Washington, D.C., 1991.

Preferable reducing agents are sodium sulfite, sodium bisulfite, sodium metabisulfite, sodium thiosulfite or other thiols, such as thioglycerol, thiourea, thioglycolic acid, cysteine and the like. Preferable antioxidants are 6-hydroxy-2,5,7,8-tetra-methylchromane-2-carboxylic acid (trolox), propyl gallate, n-propyl trihydroxybenzoate, t-butyl hydroquinone and butylated hydroxytoluene (BHT), butylated hydroxyanisole (BHA), tocopheryl acetate, ascorbyl palmitate, hydroquinone, dibutyl hydroquinone and the like.

Suitable examples of chelating agents include, but are not limited to, EDTA, citric acid, tartaric acid, organo aminophosphonic acids and organo phosphonic acid components including certain of the commercially available Dequest^{™} compounds, marketed by Monsanto. Preferred is 1-hydroxyethylene-1,1-diphosphonic acid.

Organo aminophosphonic acid is an organic compound having at least one phosphonic acid group, and at least one amino group. Suitable organo aminophosphonic acid components for use herein include the amino alkylene poly (alkylene phosphonic acids) and nitrilo trimethylene phosphonic acids. Examples of this type of organo aminophosphonic acid components include certain of the commercially available Dequest^{™} compounds, marketed by Monsanto.

Preferred are amino tri (methylene phosphonic acid) (Dequest 2006^{®}), diethylene triamine penta (methylene phosphonic acid) and hexamethylene diamine tetra (methylene phosphonic acid).

Other suitable additional heavy metal ion sequestrants for use herein include nitrilotriacetic acid and polyaminocarboxylic acids such as ethylenediaminotetracetic acid, or ethylenetriamine pentacetic acid.

Still other suitable additional heavy metal ion sequestrants for use herein are iminodiacetic acid derivatives such as 2-hydroxyethyl diacetic acid or glyceryl imino diacetic acid.

Antioxidants are included in the inventive compositions in an amount of from 0.01 to 10%, preferably from 0.1 to 5%, most preferably from 0.2 to 4%. Reducing agents are included in the inventive compositions in an amount of from 0.01 to 10%, preferably from 0.1 to 5%, most preferably from 0.2 to 4%. Chelating agents are included in the inventive compositions in an amount of from 0.01 to 1%, preferably from 0.05 to 0.5%, most preferably from 0.05 to 0.3%.

Sunscreens include those materials commonly employed to block ultraviolet light. Illustrative compounds are the derivatives of para-aminobenzoic acid (PABA), cinnamate and salicylate. For example, octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed can vary depending upon the degree of protection desired from the sun's UV radiation.

Other optional ingredients may include coloring agents, opacifiers and pigments (e.g. titanium dioxide, silica) and perfumes. Amounts of these materials may range anywhere from 0.001% up to 20% by weight of the composition, and in such a way as to keep the composition within the defined opacity limits.

### Use of the Composition

The composition according to the invention is intended primarily as a product for topical application to human skin, especially as an agent for creating or imparting radiance to the skin.

In use, a small quantity of the composition, for example from 1 to 5ml, is applied to exposed areas of the skin, from a suitable container or applicator and, if necessary, it is then spread over and/or rubbed into the skin using the hand or fingers or a suitable device.

The compositions may be designed to be left on the skin after application (i.e. leave-on) or washed off (i.e., wash-off or skin cleansing).

### Product Form and Packaging

The composition can be packaged in a suitable container to suit its viscosity and intended use by the consumer. For example, a composition can simply be stored in a non-deformable bottle or squeeze container, such as a tube or a lidded jar. A bottle may be equipped with a pump.

The invention accordingly also provides a closed container containing a cosmetically acceptable composition as herein defined.

The following specific examples further illustrate the invention, but the invention is not limited thereto.

### EXAMPLES 1 to 5

Examples of formulations according to the present invention are outlined in table 1.

**TABLE 1**

| Ingredient Trade and CTFA Name | Phase | 1 | 2 | 3 | 4 | 5 |
|---|---|---|---|---|---|---|
| Stearic acid | A | 14.9 | 14.9 | 12.9 | 17.9 | 15.7 |
| Sodium cetearyl sulfate (Anionic emulsifiers) | A | 1.0 | 1.0 | 1.5 | 1.5 | 0.5 |
| Myrj 59 (Nonionic emulsifiers) | A | 2.0 | 1.5 | 2 | 2 | 2 |
| Span 60 (Nonionic emulsifiers) | A | 2.0 | 1.5 | 2 | 2 | 2 |
| Parsol 1789 | A | 0.40 | | 0.4 | 0.4 | 0.4 |
| Propyl paraben | A | 0.10 | 0.10 | 0.10 | 0.10 | 0.10 |
| Butylated hydroxtoluene (BHT) | A | 0.1 | 0.1 | 0.1 | 0.1 | 0.1 |
| Parsol MCX | A | 0.75 | | 0.75 | 0.75 | 0.75 |
| Dimethicone | A | | 0.50 | 0.75 | | 0.75 |
| Bismuth Oxychloride | D | 0.10 | 0.05 | 0.50 | 0.10 | 0.20 |
| Water* | B | BAL* | BAL* | BAL* | BAL* | BAL* |
| EDTA | B | 0.04 | 0.04 | 0.04 | 0.04 | 0.04 |
| Pemulen TR 2 | B | | 0.10 | 0.05 | | 0.05 |
| Methyl paraben | B | 0.15 | 0.15 | 0.15 | 0.15 | 0.15 |
| Skin benefit agents | C | 3.5 | 2.0 | 2.0 | 2.0 | 2.0 |
| Humectant | C | 10 | 10 | 8 | 7 | 8 |
| TOTAL | | 100 | 100 | 100 | 100 | 100 |

| | | | | | | |
|---|---|---|---|---|---|---|
| *BAL=Balanced to 100 | | | | | | |

The formulations presented in Table 1 are prepared in the following fashion. Phase A is heated at 75°C. Phase B is heated to 75°C in a container separate from that of phase A. Thereafter the phases are combined with mixing with heat being turned off. Phase C is heated to 62°C and mixed into phases A/B at 62°C. The mixture is cooled until 40°C and then packed. Phase D may be added in either before or after cooling the mixture.

### EXAMPLES 6 - 13

Further examples of formulations according to the present invention are outlined in the table below. The formulations were prepared according to the procedure outlined with reference to the examples above.

**TABLE 2**

| Ingredients | Phase | Examples (wt. %) | | | | | | | |
|---|---|---|---|---|---|---|---|---|---|
| | | 6 acid soap base | 7 | 8 | 9 | 10 | 11 | 12 | 13 |
| Stearic acid | A | 17.9 | 17.9 | 17.9 | 17.9 | 17.9 | 17.9 | 17.9 | 17.9 |
| Sodium cetearyl sulfate (Anionic emulsifiers) | A | | 2.2 | | 1 | 1.5 | 2 | 3 | 2 |
| Myrj 59 (Nonionic emulsifiers) | A | | | 2 | 2 | 2 | 2 | 2 | 1 |
| Span 60 (Nonionic emulsifiers) | A | | | 2 | 2 | 2 | 2 | 2 | 1 |
| KOH, 22% (forms in si tu soap with stearic acid) | A | 2.20 | | | | | | | |
| Bismuth Oxychloride | C | 0.10 | 0.05 | 0.50 | 0.10 | 0.20 | 0.10 | 0.05 | 0.50 |
| | | | | | | | | | |
| Water* | B | BAL | BAL | BAL | BAL | BAL | BAL | BAL | BAL |
| Glycerin | B | 1 | 1 | 1 | 1 | 1 | 1 | 1 | 1 |

| | | | | | | | | | |
|---|---|---|---|---|---|---|---|---|---|
| *BAL=Balanced to 100 | | | | | | | | | |

### EXAMPLE 14

This example illustrates the special advantages of formulating with bismuth oxychloride to achieve skin radiance while maintaining natural skin tone.

The opacity of compositions of the present invention was compared with that of commercial color make-ups that contain bismuth oxychloride. Also, titanium coated mica was evaluated for its ability to produce a radiant effect in a skin care product upon application to skin, in terms of the opacity of compositions incorporating them. Parameters to focus on are reflectance and opacity of the plate-like material in the base formulations. The reflectance (or the difference in index of refraction) has to be large enough so as to see the effect in base formulation, but low enough as not to be sparkly. In regards to the opacity, the material cannot make the final formulation too opaque (it should be fairly translucent) so as not to create an unnatural appearance. The ideal materials will have the proper balance between these parameters. A comparison is also made using eye shadow formulations containing plate-like single crystals (bismuth oxychloride) but the formulations are opaque due to other ingredients (opacifiers) present.

A base formulation as shown in the table below was prepared as follows:
- 1.: Heat phase A to 80°C.
- 2.: Heat phase B to 75°C in a separate container.
- 3.: Add phase B to phase A and mix with heat off for 30 min.
- 4.: At 50°C add phase C and mix for 10 min.
varying amounts of mica and bismuth oxychloride were added to the base formulation, as indicated by the pigment loading in the table below, and the opacity was measured.

**TABLE 3**

| **Material Name** | **Phase** | **Wt. % in product** |
|---|---|---|
| DI (de-ionized) water/pH control | B | BALANCE |
| Disodium EDTA | B | 0.1 |
| Methylparaben NF | A | 0.2 |
| Veegum (bentonite clay) | A | 0.60 |
| Keltrol CG 1000 (xanthan gum) | A | 0.2 |
| Polymer/Silicone | A | 3.0 |
| Glycerin | B | 10.0 |
| Phenoxyethanol | B | 0.4 |
| Cetyl alcohol | B | 0.5 |
| Arlacel 60 (sorbitan stearate) | A | 1.1 |
| Myri 59 (PEG-100 stearate) | A | 0.5 |
| Emulsyny gdl (glyceryl dilaurate) | A | 0.5 |
| Pristerene 4911 (stearic acid) | A | 1.0 |
| Propyl paraben | A | 0.1 |
| Cholesterol | A | 0.5 |
| Octyl methoxycinnamate (Parsol MCX) | A | 3.0 |
| Avobenzophenone (Parsol 1789) | A | 2.0 |
| Skin Benefit Agent | C | 6.7 |
| Polymethyl methacrylate (Ganzpearl GMP0820) | B | 1.2 |
| Dermoblock OS (ethylhexyl salicylate) | B | 4.0 |

| **Material Trade Name** | **Phase** | **Wt. % in product** |
|---|---|---|
| Pecosil PAN 418 (steardiammonium hydroxypropyl panthenyl PE6-7 dimethicone phosphate chloride) | B | 4.0 |
| Dequest 2006 (aminotrimethylenephosphonic acid) | B | 0.53 |
| BHT (butylated hydroxytoluene) | A | 0.2 |
| Fragrance | C | 0.3 |
| | | |
| | | |
| PIGMENT LOADING | C | |
| | | |
| Mica (0.5% loading) | | 0.5 |
| OR | | |
| Bismuth oxychloride liquid silver (0.1 % loading) | | 0.1 |
| OR | | |
| Bismutch oxychloride liquid silver (0.5 % loading) | | 0.5 |

Methods - The opacity of formulations containing plate-like single crystals (in this case bismuth oxychloride, with a D₅₀ = 16 microns and mica with a D₅₀ = 22 microns where D₅₀ means average particle diameter) is determined using a Hunter Lab spectrocolorimeter. The procedure involves making a draw down with the formulation on a black and white draw down card. The opacity factor is determined from the ratio of L values from the black and white background portions of the draw down card. The formulations studied were 0.1% and 0.5% bismuth oxychloride, 0.5% mica, Almay Bright Eyes Color Cream Shadow Lilac Luster and its dilution to 40% with isopropanol. The formulations of the base used with the bismuth oxychloride and mica are set forth in the table above. The reflectance was evaluated visually.

### Results

The following table contains the opacity results for the formulations and materials studied:

**TABLE 6**

| Formulation | Opacity |
|---|---|
| Base Formulation | 1.3% |
| 0.5% mica in base | 1.3% |
| 0.1% bismuth oxychloride in base | 2.8% |
| 0.5 % bismuth oxychloride in base | 10.1% |
| ALMAY Bright Eyes brand, Color Cream Shadow, Lilac Luster | 97% |
| ALMAY diluted to 40% with IPA | 53% |

The mica formulation exhibited no reflectance from the plate-like crystals (the index of refraction at 1.6 is too low). For bismuth oxychloride (with an index of refraction of -2.0), the 0.1% formulation showed aesthetically pleasing "radiance" while 0.5% is a little too much (but still falls within limits).

### Conclusions

From the results above, the preferred index of refraction range for the plate-like particles is 1.8 to 2.2, as particles with lower values provide very different refractive qualities. If the value is too low, the effect is lost in the formulation (there is no visual distinction between the material and formulation). Too high and the effect will be very shiny and sparkly. The materials (plate-like crystals) added cannot increase/induce opacity in the formulation and give the product an unnatural/cosmetic appearance.

The data above show that the compositions according to the present invention are much less opaque, which permits reflective radiance whilst maintaining a natural, non-cosmetic skin tone color.

## Claims

1. A skin care or cleansing composition comprising:
a) 0.01 % to 1 % by weight of said composition of solid single-crystal, flat, plate-like particles; said particles having an index of refraction of 1.8 to 2.2; and
b) a cosmetically acceptable vehicle;
wherein said composition has an opacity of less than about 20%;
wherein said plate-like particles have a particle diameter of 10 to 30 microns;
wherein said plate-like particles are bismuth oxychloride, and wherein opacity is measured using a Hunterlab LabScan XE automated spectrophotometer with composition coatings prepared on Leneta Form 2A opacity test charts held in place on a vacuum plate, an 8-path wet film applicator used to coat a film with a wet thickness of 50.8 µm and the coatings air dried before opacity measurement.

2. A composition according to claim 1 having an opacity of less than 10 %.

3. A composition according to claim 1 or claim 2, wherein said plate-like particles have a particle thickness of 0.1 to 5 microns.

4. A composition according to any one of the preceding claims, that is a leave-on or wash-off composition.

5. A composition according to any one of the preceding claims, wherein said plate-like particles comprise 0.05 % to 0.5 % by weight of said composition.

6. A composition according to claim 5, wherein said plate-like particles comprise 0.1 % by weight of said composition.

7. A composition according to any of the preceding claims, wherein said plate-like particles are contained suspended in a polar solvent prior to incorporation in said composition.

8. A composition according to any one of the preceding claims, further comprising a skin benefit agent.

9. A composition according to claim 8, wherein the amount of said skin benefit agent is at least 0.0001% by weight of the composition.

10. A composition according to claim 8 or claim 9 wherein the skin benefit agent is selected from the group consisting of retinoids, essential fatty acids, alpha-hydroxy acids, beta-hydroxy acids, poly-hydroxy acids, skin lightening agents, and mixtures thereof.

11. A composition according to claim 10 wherein said skin benefit agent is selected from the group consisting of retinol, l.inoleic acid, glycolic acid, lactic acid, 2-hydroxyoctanoic acid, salicylic acid, ferulic acid, sebacic acid, and combinations thereof.

12. A cosmetic skin care composition according to claim 1, wherein said composition is colourless.

13. A composition according to claim 12, wherein said plate-like particles have a particle thickness of 0.1 to 5 microns.

14. A method of imparting radiant skin appearance by applying to the skin a composition in accordance with any one of the preceding claims.

## Patentansprüche

1. Hautpflege- oder -reinigungs-Zusammensetzung, umfassend:
a) 0,01 bis 1 Gew.-%, bezogen auf die Zusammensetzung, fester, flacher, plättchenartiger Einkristallpartikel; wobei die Partikel einen Brechungsindex von 1,8 bis 2,2 haben, und
b) ein kosmetisch verträgliches Vehikel;
wobei die Zusammensetzung eine Opazität von weniger als etwa 20 % hat;
wobei die plättchenartigen Partikel einen Partikeldurchmesser von 10 bis 30 Mikrometer haben;
wobei die plättchenartigen Partikel Wismutoxychlorid sind und wobei die Opazität unter Verwendung eines automatischen Hunterlab LabScan XE-Spektralfotometers mit Zusammensetzungsbeschichtungen, hergestellt an Leneta Form 2A-Opazitätstest-Charts, die an einer Vakuumplatte gehalten werden, gemessen wird, wobei ein 8-Wege-Nassfilmapplikator verwendet wird, um einen Film mit einer nassen Dicke von 50,8 µm aufzutragen, und die Beschichtungen vor einer Opazitätsmessung luftgetrocknet werden.

2. Zusammensetzung gemäß Anspruch 1, die eine Opazität von weniger als 10 % hat.

3. Zusammensetzung gemäß Anspruch 1 oder Anspruch 2, wobei die plättchenartigen Partikel eine Partikeldicke von 0,1 bis 5 µm haben.

4. Zusammensetzung gemäß einem der vorangehenden Ansprüche, die eine "Leave-on"-Zusammensetzung oder eine Zusammensetzung zum Abwaschen ist.

5. Zusammensetzung gemäß einem der vorangehenden Ansprüche, wobei die plättchenartigen Partikel 0,05 bis 0,5 Gew.-% der Zusammensetzung ausmachen.

6. Zusammensetzung gemäß Anspruch 5, wobei die plättchenartigen Partikel 0,1 Gew.-% der Zusammensetzung ausmachen.

7. Zusammensetzung gemäß einem der vorangehenden Ansprüche, wobei die plättchenartigen Partikel vor Einarbeitung in die Zusammensetzung suspendiert in einem polaren Lösungsmittel enthalten sind.

8. Zusammensetzung gemäß einem der vorangehenden Ansprüche, die außerdem ein Mittel mit günstigen Eigenschaften für die Haut umfasst.

9. Zusammensetzung gemäß Anspruch 8, wobei die Menge des Mittels mit günstigen Eigenschaften für die Haut wenigstens 0,0001 Gew.-% der Zusammensetzung ist.

10. Zusammensetzung gemäß Anspruch 8 oder 9, wobei das Mittel mit günstigen Eigenschaften für die Haut ausgewählt ist aus der Gruppe, bestehend aus Retinoiden, essentiellen Fettsäuren, alpha-Hydroxysäuren, beta-Hydroxysäuren, Polyhydroxysäuren, Haut aufhellenden Mitteln und Gemischen davon.

11. Zusammensetzung gemäß Anspruch 10, wobei das Mittel mit günstigen Eigenschaften für die Haut aus der Gruppe, bestehend aus Retinol, Linolsäure, Glykolsäure, Milchsäure, 2-Hydroxyoctansäure, Salicylsäure, Ferulasäure, Sebacinsäure und Kombinationen davon, ausgewählt ist.

12. Kosmetische Hautpflege-Zusammensetzung gemäß Anspruch 1, wobei die Zusammensetzung farblos ist.

13. Zusammensetzung gemäß Anspruch 12, wobei die plättchenartigen Partikel eine Partikeldicke von 0,1 bis 5 Mikrometer haben.

14. Verfahren, um der Haut ein strahlendes Aussehen zu verleihen, indem eine Zusammensetzung gemäß einem der vorangehenden Ansprüche auf die Haut aufgetragen wird.

## Revendications

1. Composition nettoyante ou de soin de la peau comprenant :
a) de 0,01 % à 1 % en poids de ladite composition de particules de type plaque, plates, monocristallines, solides ; lesdites particules ayant un indice de réfraction de 1,8 à 2,2 ; et
b) un véhicule cosmétiquement acceptable ;
➢ ladite composition ayant une opacité inférieure à environ 20 % ;
➢ lesdites particules de type plaque ayant un diamètre de particule de 10 à 30 µm ;
➢ lesdites particules de type plaque étant de l'oxychlorure de bismuth, et l'opacité étant mesurée en utilisant un spectrophotomètre automatisé Hunterlab LabScan XE avec des revêtements de composition préparés sur des cartes de test d'opacité Leneta Form 2A maintenues en place sur une plaque sous vide, un applicateur de film humide à 8 voies étant utilisé pour déposer un film ayant une épaisseur humide de 50,8 µm, et les revêtements étant séchés à l'air avant la mesure de l'opacité.

2. Composition selon la revendication 1, ayant une opacité inférieure à 10 %.

3. Composition selon la revendication 1 ou la revendication 2, dans laquelle lesdites particules de type plaque ont une épaisseur de particule de 0,1 à 5 µm.

4. Composition selon l'une quelconque des revendications précédentes, qui est une composition sans rinçage ou à rincer.

5. Composition selon l'une quelconque des revendications précédentes, dans laquelle lesdites particules de type plaque représentent de 0,05 % à 0,5 % en poids de ladite composition.

6. Composition selon la revendication 5, dans laquelle lesdites particules de type plaque représentent 0,1 % en poids de ladite composition.

7. Composition selon l'une quelconque des revendications précédentes, dans laquelle lesdites particules de type plaque sont contenues en suspension dans un solvant polaire avant leur incorporation dans ladite composition.

8. Composition selon l'une quelconque des revendications précédentes, comprenant en outre un agent bénéfique pour la peau.

9. Composition selon la revendication 8, dans laquelle la quantité dudit agent bénéfique pour la peau est d'au moins 0,0001 % en poids de la composition.

10. Composition selon la revendication 8 ou la revendication 9, dans laquelle l'agent bénéfique pour la peau est choisi dans le groupe constitué par les rétinoïdes, les acides gras essentiels, les alpha-hydroxyacides, les bêta-hydroxyacides, les poly-hydroxyacides, les agents éclaircissant la peau, et des mélanges de ceux-ci.

11. Composition selon la revendication 10, dans laquelle ledit agent bénéfique pour la peau est choisi dans le groupe constitué par le rétinol, l'acide linoléique, l'acide glycolique, l'acide lactique, l'acide 2-hydroxyoctanoïque, l'acide salicylique, l'acide férulique, l'acide sébacique, et des combinaisons de ceux-ci.

12. Composition cosmétique de soin de la peau selon la revendication 1, ladite composition étant incolore.

13. Composition selon la revendication 12, dans laquelle lesdites particules de type plaque ont une épaisseur de particule de 0,1 à 5 µm.

14. Procédé conférant une apparence rayonnante à la peau par l'application sur la peau d'une composition selon l'une quelconque des revendications précédentes.
